Europäisches Patentamt

European Patent Office

Office européen des brevets

(11) Publication number: **0 366 816**
**A1**

# (12) EUROPEAN PATENT APPLICATION

(21) Application number: 88118166.3

(22) Date of filing: 31.10.88

(51) Int. Cl.5· **C12N 15/16 , C12P 21/02**

(43) Date of publication of application:
09.05.90 Bulletin 90/19

(84) Designated Contracting States:
AT BE CH DE ES FR GB GR IT LI LU NL SE

(71) Applicant: RORER BIOTECHNOLOGY INC.
680 Allendale Road
King of Prussia Pennsylvania(US)

Applicant: JEWISH HOSPITAL OF ST. LOUIS
216 South Kingshighway
St. Louis, MO 63110(US)

(72) Inventor: Tong, Benton D.
40 N. Kingshighway Apt. 6C
St. Louis Missouri(US)
Inventor: Deuel, Thomas F.,MD
81 Aberdeen Place
Clayton Missouri(US)
Inventor: Jaye, Michael C.
3017 South Second Street
Arlington Virginia(US)
Inventor: Drohan, William N.
5232 Perth Court
Springfield Virginia(US)

(74) Representative: Hansen, Bernd, Dr.rer.nat. et
al
Hoffmann, Eitle & Partner Patentanwälte
Arabellastrasse 4 Postfach 81 04 20
D-8000 München 81(DE)

(54) Recombinant platelet-derived growth factor A-chain derived from endothelial cells.

(57) A PDGF A-chain clone has been isolated from a human umbilical vein endothelial cell-derived cDNA library, the DNA sequence of which identifies a gene with a 69 base pair deletion in comparison to a previously described cDNA clone from a malignant glioma. It is significant that this clone is derived from a non-transformed normal human cell rather than from a malignant glioma. Therefore, the PDGF A-chain encoded by the gene isolated in this invention represents the normal PDGF A-chain, and variant sequences thereof will also be included.

Accordingly, the present invention provides human PDGF A-chain essentially free of other proteins of human origin. The invention further provides replicable expression vectors incorporating a DNA sequence encoding PDGF A-chain and a self-replicating host cell system transfected thereby resulting in the production of PDGF A-chain or functional fragments thereof in useful quantities and purities. In addition, the PDGF A-chain thereby produced is free of contaminants associated with the protein in its non-recombinant cellular environment.

# RECOMBINANT PLATELET-DERIVED GROWTH FACTOR A-CHAIN DERIVED FROM ENDOTHELIAL CELLS

This invention relates to the platelet-derived growth factor (PDGF) A-chain, or variants thereof, derived from a non-transformed human cell and its DNA-directed synthesis, via the application of recombinant DNA technology.

The purification of human platelet-derived growth factor (PDGF) to homogeneity has been reported. The reported molecular weights have been in the range 27,500 - 35,000 daltons. Human PDGF is a strongly cationic and hydrophobic protein. It is remarkably resistant to heat and chemical denaturants, perhaps due to stabilization of the protein structure by many disulphide bonds since PDGF remains susceptible to reducing agents. Studies over the past 13 years have established PDGF as a growth factor of major interest, and it is thought to play a role in normal tissue repair processes in conjunction with the platelet release response to blood vessel injury.

PDGF is strongly mitogenic for cells of mesenchymal origin. In addition, PDGF was shown to have an important biological activity in vitro as a strong chemo-attractant for human inflammatory cells, including monocytes, neutrophils, fibroblasts, and smooth muscle cells. The combined biological properties of PDGF as a potent mitogen and chemo-attractant suggest PDGF as a key agent involved in several of the initial events in the pathogenesis of human atherosclerosis, as well as affecting inflammation and wound healing. PDGF has also been implicated in bone resorption following abnormal platelet release and in the growth of tumor cells.

Peptide growth factors may play important roles in the body's response to injury by promoting wound healing. Epidermal growth factor, present in saliva, is thought to accelerate healing of cutaneous injuries in mice as they lick their wounds. Topical application of epidermal growth factor has been shown to accelerate epidermal regeneration of middermal skin injuries, corneal abrasions, and increased tensile strength of corneal incisions. In addition, transforming growth factor and vaccinia growth factor have recently been shown to accelerate epithlial regeneration in middermal thermal injuries. These findings suggest that the topical application of growth factors may be useful in accelerating the healing of partial thickness abrasion or burn injuries. The unique properties of PDGF and its interrelation with cells of mesenchymal origin may offer other pharmaceutical applications in response to inflammation or atherosclerosis. Ultimately, the differences between the non-transformed derived PDGF A-chain and other variants derived from cellular transformation, may prove significant to the identification or control of the transforming function of the PDGF gene.

Human platelet-derived growth factor (PDGF) consists of a heterodimer or a mixture of homodimers of polypeptide chains, described as A and B by Deuel et al. (1984) Blood 64, 951-950. However, the dimeric structure is functionally important, since reduction or exposure to proteolytic agents irreversibly destroys the mitogenic activity of PDGF. High-affinity cell-surface PDGF receptors, located in connective tissue-derived cells, respond to PDGF with tyrosine specific kinase activity leading to the phosphorylation of various cellular proteins and to receptor autophosphorylation. Other cellular responses to PDGF include increased cytoplasmic calcium concentration, activation of protein kinase C, cytoplasmic alkalinization, reorganization of actin filaments, specific gene expression including cellular oncogenes, and DNA synthesis.

The PDGF B-chain precursor is encoded by the c-sis proto-oncogene, the cellular homologue of the transforming gene v-sis of simian sarcoma virus (SSV). A comparison of the amino terminus portion of the known sequence of the PDGF B-chain with that of the v-sis product reveals a region of 93% identity over a 109 amino acid residue stretch. B-chain mRNA has been detected in several human tumor cell lines, as well as in certain normal cell types including endothelial cells, placental cytotrophoblasts and activated macrophages.

The primary translation product of the v-sis gene was shown to be modified by dimerization and proteolytic processing at the N- and C- termini, yielding a product which resembles a dimer of PDGF B-chains and is recognized by anti-PDGF antibodies. Based on competition with $^{125}$I-PDGF for receptor binding, secretion of the PDGF-like product appears to stimulate the growth of SSV-transformed cells through PDGF cell surface receptors. Cells transformed by SSV have been shown to contain and release a PDGF agonist activity. In addition, SSV-transformed human fibroblasts are morphologically indistinguishable from PDGF-stimulated cells, and the transformed phenotype is abolished by the addition of anti-PDGF antibodies to the culture medium.

It has been established that PDGF B-chain, i.e., the PDGF-like factor from SSV-transformed cells, can form an active mitogen. The mitogenic activity of the B-chain is also apparent in porcine PDGF which is constructed exclusively of dimers similar to human B-chain. PDGF A-chains also may have biological activity based on studies of a PDGF-like mitogen released from a human osteosarcoma cell line, U-2 OS. The osteosarcoma-derived growth factor (ODGF) was found to bind to the PDGF receptor, in addition to having the same functional and immunological characteristics as PDGF. Recent studies have shown the protein to be a homodimer of a polypeptide chain that displays a chemical fragmentation pattern, chromatographic behavior and N-terminal amino-acid sequence identical to that of the PDGF A-chain. Therefore, apparently the formation of homodimers of either PDGF A or B genes may lead to the production of PDGF agonist activity since both chains have been shown to interact with the PDGF receptor.

Although encoded by individual genes, significant homology exists between the PDGF A- and B-chain. The A- and B-chain precursors share 40% homology at the amino acid level. Notably greater homology, 56%, exists within amino acid region 89-181 within the mature chains. The two constituent chains of human PDGF are encoded by genes located on difference chromosomes. The human PDGF B-chain (c-sis) gene has been mapped to the long arm of chromosome 22, while PDGF A-chain has been localized to chromosome 7. However, these proteins share essentially no sequence homology at their respective C-termini.

Genes coding for polypeptides such as PDGF A-chain or fragments of PDGF A-chain may be cloned by incorporating a DNA fragment coding for the polypeptide into a recombinant DNA vehicle (e.g., prokaryotic or eukaryotic vectors) and transforming a suitable host. Such recombinant DNA techniques have now become well known and are described in Methods In Enzymology, Colowick et al., Vol. 63, 68 (1978), vol. 100 and 101 (1983) Academic Press, New York, NY., and the references cited therein, which are incorporated herein by reference. An extensive technical discussion embodying most commonly used recombinant DNA methodologies can be found in Maniatis et al., Molecular Cloning: A Laboratory Manual (1982), Cold Spring Harbor Laboratory, Cold Spring Harbor, NY.

One way of obtaining a DNA fragment encoding a desired polypeptide, such as PDGF A-chain, is via cDNA cloning. In this process, messenger RNA (mRNA) is isolated from cells known or suspected of producing the desired protein. Through a series of enzymatic reactions, the mRNA population of the cells is copied into a complementary DNA (cDNA). The resulting cDNA is then inserted into cloning vehicles and subsequently used to transform a suitable prokaryotic or eukaryotic host. The resultant gene "library" is comprised of a population of transformed host cells, each of which contain a single gene or gene fragment. The entire library, therefore, provides a representative sample of the coding information present in the mRNA mixture used as a starting material.

Gene libraries are screened using specific nucleic acid or antibody probes. Nucleic acid probes are useful for locating cDNAS by hybridization and autoradiographic techniques. This approach, however, requires previous knowledge of at least a portion of the protein's amino acid or DNA sequence. Alternatively, methods have been developed to identify specific clones by probing recombinant gene libraries with antibodies specific for the encoded protein of interest. This method can only be used with "expression vector" cloning vehicles since elaboration of the product protein is required. An example of this is the bacteriophage lambda gtll system.

Once isolated, these cDNAS can be genetically engineered. Gene fragments can be assembled into complete genes. Several methods are currently in use for delivering defined foreign DNA segments into host vector cells. Following the addition of necessary genetic regulatory elements, a vector can accurately express heterologous genes, such as PDGF A-chain, from the cell line selected for production. The proteins encoded by these engineered gene fragments may not be found in nature, yet they may have significant utility in treating undesirable physiological conditions.

A PDGF A-chain clone has been isolated from a human umbilical vein endothelial cell-derived cDNA library, the DNA sequence of which identifies a gene with a 69 base pair deletion in comparison to a previously described cDNA clone from a malignant glioma. It is significant that this clone is derived from a non-transformed normal human cell rather than from a malignant glioma. Therefore, the PDGF A-chain encoded by the gene isolated in this invention represents the normal PDGF A-chain, and variant sequences thereof will also be included.

The present invention has made it possible to provide readily available quantities of human PDGF A-chain from a non-transformed source. The functional PDGF A-chain will be sufficient for identification and characterization of the protein prior to pharmaceutical application. This has been achieved by the application of recombinant DNA methodolgy for the construction of cloning vehicles encoding the human PDGF A-chain protein, screening of positive recombinant cells, and isolation/purification procedures for recovering human PDGF A-chain essentially free of other proteins of human origin. Similar cloning vehicles have been constructed encoding other unique human proteins.

Accordingly, the present invention provides human PDGF A-chain essentially free of other proteins of human origin. The invention further provides replicable expression vectors incorporating a DNA sequence encoding PDGF A-chain and a self-replicating host cell system transfected thereby resulting in the production of PDGF A-chain or functional fragments thereof in useful quantities and purities. In addition, the PDGF A-chain thereby produced is free of contaminants associated with the protein in its non-recombinant cellular environment.

The PDGF A-chain encoding replicable expression vector is made by preparing a double-stranded complementary DNA (ds-cDNA) preparation representative of PDGF A-chain mRNA, and incorporating the ds-cDNA into replicable expression vectors. The preferred mode of recovering the PDGF A-chain comprises reacting the proteins expressed by the recombinant host system with a reagent composition comprising at least one binding protein specific for PDGF A-chain used in the recovery step.

Description of the Drawings

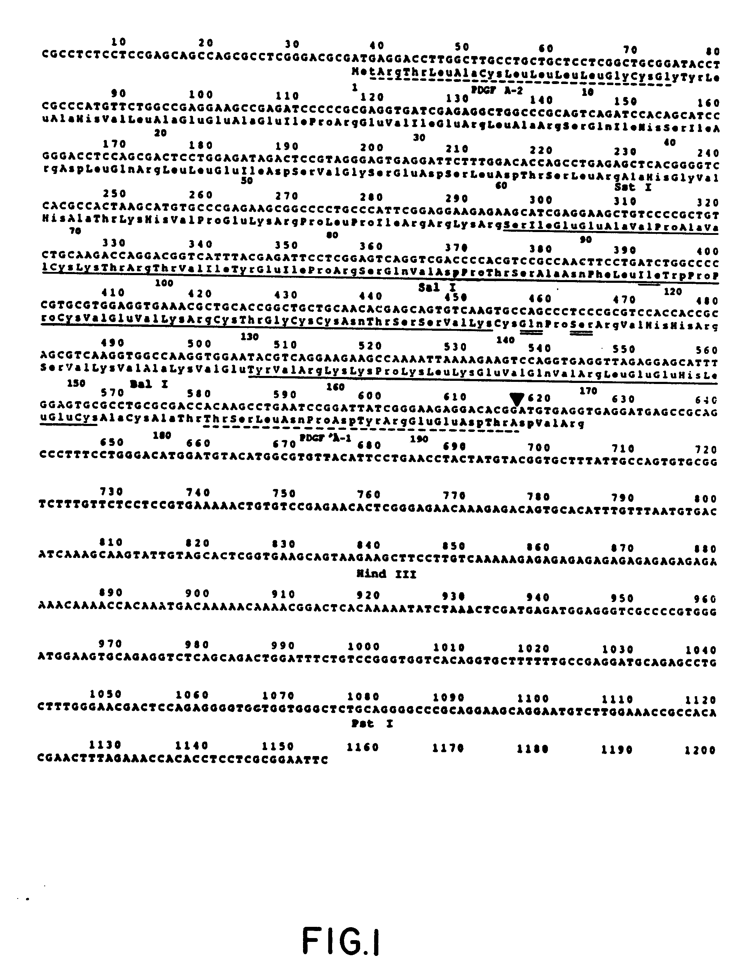

Figure 1. Sequence of A-chain clone BT-1

The cDNA from each clone which hybridized to the PDGF A-1 and PDGF A-2 probes was isolated from the recombinant lambda gtll phage and subcloned into the EcoRI site of pGem2 (Promega Biotec). Each clone was sequenced by the dideoxy method using either the SP6 and T-7 primers from Promega Biotec or 17-22 base pair long oligonucleotide primers. The predicted amino acid sequence of the largest open reading frame is shown. The amino acids which are identical to those previously determined by amino acid sequencing are underlined by solid lines. The amino acid residues that differ from those previously determined by amino acid sequencing are underlined twice. This indicates the site of the 69 base pair difference between BT-1 and D-1. The --- line indicates the location of either of PDGF A-1 probe or the PDGF A-2 probe.

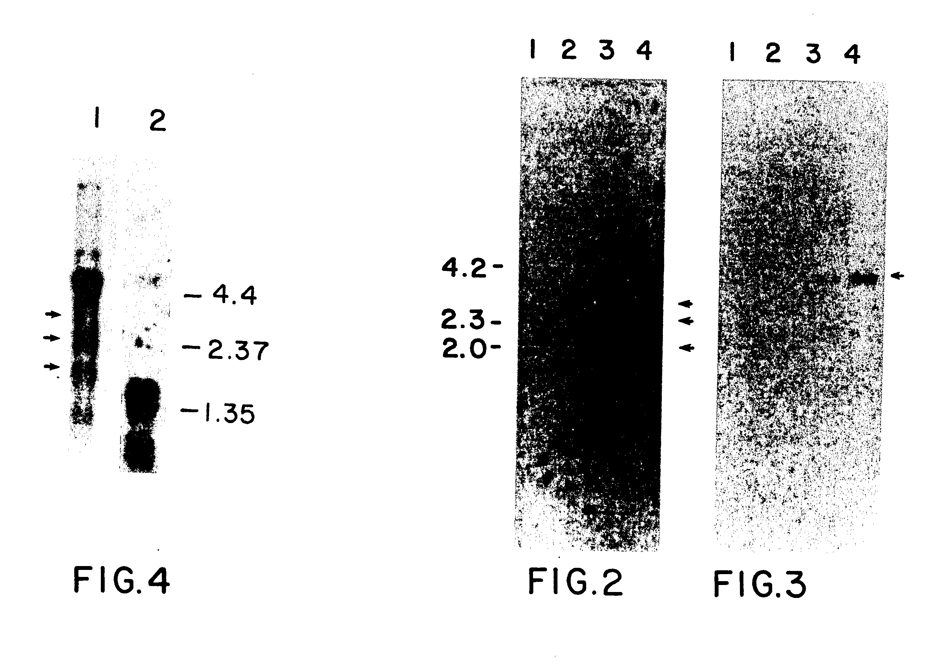

Figure 2. Modulation of the PDGF A-chain mRNA in endothelial cells.

Endothelial cell mRNA was isolated, fractionated, and blotted for Northern analysis as described in the Recombinant and Screening Methodology. The filter was hybridized to nick-translated BT-1, then exposed to Kodak XAR-5 film with a Dupont Cronex intensifying screen overnight at -70° C. Arrows indicate the positions of the 3 PDGF A-chain mRNAS. HindIII digested lambda DNA was used for size markers. Lane 1: 22 ug yeast tRNA; lane 2: 5 ug poly(A) containing mRNA from rat promegakaryoblast cells; lane 3: 5 ug of poly(A) containing mRNA from organized human umbilical vein endothelial cells; lane 4: 5ug of poly (A) containing mRNA from monolayer human umbilical vein endothelial cells.

Figure 3. Modulation of PDGF B-chain mRNA in endothelial cells.

The blot used in the Northern analysis in Figure 2 was stripped using 0.1 M NaOH and rehybridized to a nick translated v-sis probe. The resulting filter was subjected to autoradiography as above. Arrows indicate the positions of the PDGF B-chain mRNA.

Figure 4. Absence of 69 pb insert in human umbilical vein endothelial cell poly(A) containing RNA.

Five ug of poly(A) containing mRNA from monolayer human umbilical vein endothelial cells were electrophoresed and transferred to nitrocellulose as described above. The filters were then hybridized at 45° C. The probe was either a 32-mer deletion-flanking $^{32}$p-labeled oligonucleotide that would hybridize only to mRNA not containing the insert (lane 1), or to a 32-mer $^{32}$p-labeled oligonucleotide derived from the nucleotide sequence of the deleted portion (lane 2). The resulting filter was subjected to autoradiography as above. Arrows indicate the positions of the PDGF A-chain mRNA. An RNA ladder (Bethesda Research Laboratories) was used for size markers.

Definitions

As used hereafter, "PDGF A-chain" denotes the A-chain of human platelet-derived growth factor or its fragments produced by cell or cell-free culture systems in biologically active forms having the capacity to affect cellular growth.

Different alleles of the PDGF A-chain may exist in nature. These variations may be characterized by difference(s) in the nucleotide sequence of the structural gene coding for proteins of identical biological function. In addition, the location and degree of possible glycosylation, as well as other post-translation modifications may vary, and will depend, to a degree, upon the nature of the host and environment in which the protein is produced. It is possible to produce analogs having single or multiple amino acid substitutions, deletions, additions, or replacements. All such allelic variations, modifications, and analogs resulting in derivatives of the PDGF A-chain, which retain the biologically active properties of the native PDGF A-chain, are included within the scope of this invention.

"Expression vectors" refer to vectors which are capable of directing the expression of the DNA sequences contained therein, where such sequences are linked to other regulatory sequences capable of effecting their expression. These expression vectors must replicate in the host organisms or cell culture systems either as episomes, bacteriophage, or as an integral part of the chromosomal DNA.

"Recombinant host cells" refer to cells which have been transformed with vectors constructed and described herein using recombinant DNA techniques. As defined herein, PDGF A-chain may be produced as a consequence of this transformation. PDGF A-chain or its fragments produced by such cells may also be referred to as "recombinant PDGF A-chain".

Size units for DNA and RNA are often abbreviated as follows: bp = base pair; kb = kilo (one thousand) base pair. For proteins we abbreviate: D = dalton; kD = kilodalton. Volumes are measured in ml = milliliters or ul = microliters, mass in g = grams, mg = millilgrans, or ug = micrograms, concentrations in molarity; M = molar, mM = millimolar, or uM = micromolar. Temperatures are always given in degrees Celsius. Units of radioactivity are in Ci = curies or mCi = millicuries with measurements in cpm = counts per minute.

## Host Cell Cultures and Vectors

Recombinant vectors and methodology disclosed herein are suitable for use in host cells, covering a wide range of prokaryotic and eukaryotic organisms. In general, prokaryotes are preferred for the cloning and amplification of DNA sequences and in the construction of vectors. For example, E. coli K12 strain HB101 (ATCC No. 33694) is particularly useful. Other microbial strains may be used. Vectors containing replication and control sequences which are derived from species compatible with the host cell are used in connection with these hosts. The vector ordinarily carries an origin of replication and may also contain control elements which can be used by the vector for expression of its own proteins. Suitable expression vectors may contain termination signals which provide for the polyadenylation and termination of the mRNA transcript of the cloned gene.

Eukaryotic microbes or cell lines derived from multicellular organisms may also be used as hosts. In principle, any such cell culture is usable, whether from a vertebrate or invertebrate source. However, interest has been greatest in vertebrate cells, and propagation of vertebrate cells in tissue culture has become a routine procedure in recent years.

Expression vectors for such cells ordinarily include an origin of replication, a promoter located in front of the gene to be expressed, RNA splice sites (if necessary), a polyadenylation site, and transcriptional termination sequences.

The host cell is engineered to express a recombinant human PDGF A-chain peptide, identical in its primary amino acid sequence to native human PDGF A-chain.

## Recombinant and Screening Methodology

The procedures below are but some of a wide variety of well-established procedures to produce specific regents useful in the process of this invention.

## 1. Preparation of Total RNA

Total RNA (messenger, ribosomal, and transfer) was extracted from human umbilical vein endothelial cells essentially as described by Chirgwin et al. [Biochemistry 18:5294 (1979)]. Tissues were homogenized in 10 volumes of a solution containing 4 M guanidine thiocyanate, 25 mM sodium citrate at pH 7.0, 10 mM ethylenediaminetetraacetic acid, (EDTA) pH 7.4, 0.5% N-laurylsarcosine, and 0.1 M 2-mercaptoethanol. The homogenate was centrifuged at 6,000 rpm in a Sorvall GSA rotor for 15 minutes at 10° C. to clear debris. The homogenate was adjusted to pH 5.0 by the addition of 1M acetic acid and the RNA precipitated overnight with 0.75 volumes of ethanol at -20° C overnight. RNA was collected by centrifugation and the above guanidine hydrochloride precipitation step was repeated using half the previous volume. After centrifugation, the RNA pellet was dissolved in 25mM EDTA pH 7.0 and extracted with phenol: chloroform (1:1). The aqueous layer was adjusted to 0.2M NaCl, and precipitated by the addition of 2.5 volumes of ethanol at -20° C overnight. The RNA was collected by centrifugation at 10,000 rpm for 20 minutes in a Beckman JS-13 swinging bucket rotor at 0° C. The pellet was redissolved in TE buffer (10mM Tris pH 9.0, 0.1mM EDTA), adjusted to 0.2M sodium chloride, and reprecipitated with 2.5 volumes of ethanol at -20° C overnight. The RNA was then dissolved in sterile water to the desired concentration as determined by spectrophotometric readings at 260 nm, and maintained at -20° C.

## 2. Preparation of Poly(A)-containing RNA

The total RNA precipitate, prepared as described above, was dissolved at a concentration of

40 $A_{260}$ units in 20mM 4-(2-hydroxyethyl)-1-piperazineethanesulfonic acid (HEPES) buffer (pH 7.2) containing 10mM EDTA and 1% SDS, heated at 65°C for 10 minutes, then quickly cooled to 25°C. The RNA solution was then diluted with an equal volume of water, and NaCl was added to bring the final concentration to 300 mM NaCl. Samples containing up to 240 $A_{260}$ units of RNA were chromatographed on poly(U)-Sepharose using standard procedures. Poly(A)-containing RNA was eluted with 90% formamide containing 1 mM HEPES buffer (ph 7.2), and 2 mM EDTA. The eluate was adjusted to 0.24 M NaCl, and the RNA was precipitated by 2.5 volumes of ethanol at 0°C.

### 3. cDNA Probes and Nick Translation

Nick translation reactions were performed using 1 ug of each cDNA in a reaction volume of 50 ul. The reaction mixture contained the 1X nick translation buffer (50mM Tris pH 7.2, 10mM $MgSO_4$, 0.1mM dithiothreitol, 50 ug/ml bovine serum albumin), 0.1mM each of unlabeled dATP, dGTP, and dTTP, 80 uCi $^{32}$P-dCTP (specific activity = 1mCi/$1.25X10^{-9}$ moles) New England Nuclear), $5X10^{-5}$ ug deoxyribonuclease, and 10 unites of E. coli DNA polymerase I (Boehringer-Mannheim). The reaction was incubated at 16°C for 1 hour, then the cDNA was separated from the unincorporated deoxynucleotide triphosphates by chromatography on a Sephadex G-50 mini-spin column (Cooper Biomedical). The $^{32}$P-labeled DNA had a specific activity in excess of $10^7$ cpm/ug.

### 4. Construction of cDNA Clones in Lambda gtII

The poly(A)-enriched RNA isolated from human umbilical vein endothelial cells (20 ug) was copied into ds-cDNA with reverse transcriptase and DNA polymerase I. The ds-cDNA was desalted on a Sephadex G-50 column and the void volume fractions further purified on an Elutip-D column (Schleicher & Schuell) following the manufacturer's directions. The ds-cDNA was made blunt-ended by incubation with SI nuclease. The reaction mixture consisted of 0.2 M sodium acetate (pH 4.5), 0.4 M sodium chloride, 2.5 mM zinc acetate and 0.1 unit of SI nuclease per nanogram of ds-cDNA, made to a final reaction volume of 100 ul. The ds-cDNA, was incubated at 37°C for one hour, extracted with phenol:chloroform, and then desalted on a Sephadex G-50 column as described above.

The ds-cDNA was then treated with EcoRI methylase and DNA polymerase I (Klenow) using reaction conditions described in Maniatis above. The cDNA was again desalted on a Sephadex G-50 as described above, Elutip purified, and then ligated to 0.5 ug of phosphorylated EcoRI linkers using $T_4$ DNA ligase. The mixture was cleaved with EcoRI and fractionated on an 8% polyacrylamide gel in Tris-borate buffer. DNA with a size greater than 1 kilobase was eluted from the gel, recovered by binding to an Elutip-D column, eluted with 1 M NaCl and then collected by ethanol precipitation.

The DNA fragments were then inserted into EcoRI-cleaved and phosophatase-treated lambda gtII using $T_4$ DNA ligase to produce a library of approximately 1.5 million phage of which about 50% contain inserts (i.e., 0.75 million clear plaques on X-gal plates). The library was amplified by producing plate stocks at 42°C on E. coli Y1088 [supE, supF, metB, trpR, hsdR⁻, hsdm⁻, tonA21, strA, lacU169 (proC::Tn5) (pMC9)] (ATTC. No. 37195). The titer of the amplified library was determined to be about $1X10^8$ pfu/ml.

### 5. Idenfification of Clones Containing PDGF A-Chain.

Eighteen cDNA clones were isolated from a lambda gtII cDNA library constructed from human umbilical vein endothelial cell mRNA based upon hybridization to the PDGF A-1 probe. Clones which also hybridized with the PDGF A-2 probe were further characterized. The inserts ranged from 900 to 1100 bp. These were subcloned into the EcoRI site of pGem2 and sequenced using the Gem Seq ds DNA sequencing system. The sequence of the cloned DNA was then verified as PDGF A-chain by comparison to the known amino acid sequence of PDGF A-chain.

### Example

A cDNA library was constructed using mRNA from monolayer human umbilical vein endothelial (HUVE) cells. Two 36-bp probes (PDGF A-1, an internal sequence specific probe, and PDGF A-2, a N-terminus probe) were constructed based upon the nucleic acid sequence of the A-chain clone (D-1). $4 X 10^6$ phage were plated on E. coli Y1088 and incubated at 42°C for 6 hours. The phage were transferred to nitrocellulose and denatured in 0.5 M NaOH and baked at 80°C in vacuum for 2 hours. The filters were prehybridized in 5 X SSPE (1 X SSPE = 0.18 M NaCl, 0.01 M $NaPO_4$, pH 7.2, 1 mM EDTA) and 1% Blotto 0.25% non-fat dry milk) overnight at 56°C and hybridized to a kinased, 36-mer oligonucleotide A-1 ($1 X 10^6$ cpm/ml) overnight. The filters were washed twice in 2X SSC (1X SSC = 0.15 M NaCl, 0.015 M sodium citrate, pH 7) 0.1% SDS for 10 minutes at 50°C and twice in 1X SSC, 0.1% sodium dodecyl sulfate (SDA) for 30 minutes at 50°C.

Eighteen clones hybridized to the PDGF A-1 probe. These clones were further tested for hybridization to the PDGF A-2 probe in the same manner as was used for the PDGF A-1 probe. Four of the 18 clones also hybridized to the PDGF A-2 probe. Restriction mapping indicated that two of the four clones contained the same insert. The insert sizes ranged from 900 bp (BT-1) to about 1100 bp (BT-4). The clones were subcloned into the EcoRI site of pGem2. The T7 or SP6 primers of pGem2 were used for "preliminary sequencing" prior to sequencing by the Gem Seq ds DNA sequencing system [Chen, E. and Seeburg, P. DNA 4:165 (1985)]. When compared to a cDNA clone (D-1) isolated from a transformed cell line [Betsholtz, C. et al., (1986) Nature 320, 695-699], BT-1 began at nucleotide 350 and continued approximately 250 bp beyond the 3' end of the D-1 clone (Figure 1). The sequence of the 38 bp 5'-translated region of BT-1 is identical to that observed in clone D-1. BT-1 was sequenced completely and the predicted amino acid sequence determined. The protein sequence predicted by the nucleotide sequence of BT-1 agrees with that determined by amino acid sequencing of the PDGF A-chain except at amino acids 119, 141, and 143, which were predicted to be Ile, Gln, and Ser instead of the previously reported Val, Arg, and Thr. The predicted protein sequence from BT-1 agrees with that predicted by the sequence of D-1, indicating that earlier protein sequencing at these residues probably was in error.

BT-1 and the other two endothelial cell derived clones, BT-2 and BT-3, contained a DNA sequence that predicted a C-terminus different from that of D-1. A sequence of 69 base pairs that was present in the sequence of the D-1 cDNA clone isolated from the transformed cell (base pairs 967-1036) was absent in the sequences of BT-1, BT-2, and BT-3. This deletion removed the final 18 C-terminal amino acids as compared to D-1 and inserted 3 different amino acids. Thus, the deletion eliminated the stretch of Arg and Lys residues at the C-terminus and changed the overall charge of the A-chain from +11 to +4. The predicted molecular weight of the unprocessed peptide is 22,252. At nucleotide 243, in clone BT-1 a C was found instead of the T in D-1. Since both CAT and CAC code for histidine, no change in the amino acid sequence will occur. In addition, an insertion consisting of (GA)6 was found in the 3' untranslated region beginning at 868 in BT-1.

The cDNA insert of BT-1 was used to measure the steady state levels of A-chain transcripts as a function of endothelial cell differentiation. Hybridization was observed to transcripts of 2.8, 2.3, and 2.0 kb. The level of hybridization in endothelial cells derived from monolayers was increased up to two fold relative to the levels observed in endothelial cells organized into the tube-like structures (Figure 2). In contrast, the level of B-chain (c-sis) transcripts in monolayer HUVE cells was increased approximately five fold when compared to organized HUVE cells.

PDGF A-chain mRNA containing the 69 bp sequence that was present in the malignant glioma-derived A chain clone D-1, may be present in endothelial cells despite the isolation of only "deleted" clones. To test this possibility, oligonucleotide probes were utilized that could differentiate D-1 and BT-1. Probe 1 consists of a 32 bp sequence that spans the region of the deletion, flanking the 69 bp insert (base pairs 601-632) with 16 nucleotides on either side. Probe 2 consists of 32 bp that are complimentary to part of the 69 bp insert of D-1 (base pairs 1004-1036) as designated by Betsholtz et al. above. The specificity of the probes was determined by hybridization to BT-1 and D-1 cDNA. The insert-specific probe hybridized only to D-1, while the deletion spanning probe recognized both BT-1 and D-1. No hybridization was observed to the 2.8, 2.3, or 2.0 kb A-chain messages for the insert specific probe, whereas there was hybridization observed with the probe that spanned the insertion (Figure 4). This suggests that the PDGF A-chain synthesized by endothelial cells is structurally different from that made by a transformed cell line. Differences in the primary sequence and the charge of the protein derived from endothelial cells as compared to the transformed cell-derived PDGF A-chain, implies that these two forms of the PDGF A-chain may be functionally different.

## Claims

1. Platelet-derived growth factor A-chain derived from a non-transformed mammalian cell.

2. A cDNA clone comprising the coding sequence for the platelet-derived growth-factor A-chain derived from a non-transformed mammalian cell according to Claim 1.

3. A platelet-derived growth-factor A-chain according to Claim 1 derived from human umbilical vein endothelial cell.

4. A self-replicating expression vector capable of expressing in an in vitro culture system a recombinant platelet-derived growth-factor A-chain according to Claim 1.

5. A platelet-derived growth-factor A-chain according to Claim 1 as illustrated in Figure 1.

6. A process for preparing platelet-derived growth-factor A-chain derived from a non-transformed mammalian cell comprising expressing in a self-replicating host cell system a replicable expression vector incorporating a DNA sequence encoding said platelet-derived growth-factor A-chain.

Claims for the following Contracting States: ES, GR

1. A process for preparing platelet-derived growth-factor A-chain derived from a non-transformed mammalian cell comprising expressing in a self-replicating host cell system a replicable expression vector incorporating a DNA sequence encoding said platelet-derived growth-factor A-chain.

2. The process according to Claim 1 wherein the DNA is a cDNA clone comprising the coding sequence for platelet-derived growth-factor A-chain derived from a non-transformed mammalian cell.

3. The process according to Claim 1 wherein platelet-derived growth-factor A-chain is derived from human umbilical vein endothelial cell.

4. The process according to Claim 1 wherein the self-replicating vector is capable of expressing in an in vitro culture system a recombinant platelet-derived growth-factor A-chain.

5. The process according to Claim 1 wherein the platelet-derived growth-factor A-chain is as illustrated in Figure 1.

```
         10        20        30        40        50        60        70        80
CGCCTCTCCTCCGAGCAGCCAGCGCCTCGGGACGCGATGAGGACCTTGGCTTGCCTGCTGCTCCTCGGCTGCGGATACCT
                                   MetArgThrLeuAlaCysLeuLeuLeuLeuGlyCysGlyTyrLe
                                   1                    PDGF A-2              10
         90       100       110       120       130       140       150       160
CGCCCATGTTCTGGCCGAGGAAGCCGAGATCCCCCGCGAGGTGATCGAGAGGCTGGCCCGCAGTCAGATCCACAGCATCC
uAlaHisValLeuAlaGluGluAlaGluIleProArgGluValIleGluArgLeuAlaArgSerGlnIleHisSerIleA
              20                                              30
        170       180       190       200       210       220       230       240
GGGACCTCCAGCGACTCCTGGAGATAGACTCCGTAGGGAGTGAGGATTCTTTGGACACCAGCCTGAGAGCTCACGGGGTC
rgAspLeuGlnArgLeuLeuGluIleAspSerValGlySerGluAspSerLeuAspThrSerLeuArgAlaHisGlyVal
                        50                        60                    Sst I
        250       260       270       280       290       300       310       320
CACGCCACTAAGCATGTGCCCGAGAAGCGGCCCCTGCCCATTCGGAGGAAGAGAAGCATCGAGGAAGCTGTCCCCGCTGT
HisAlaThrLysHisValProGluLysArgProLeuProIleArgArgLysArgSerIleGluGluAlaValProAlaVa
     70                              80
        330       340       350       360       370       380       390       400
CTGCAAGACCAGGACGGTCATTTACGAGATTCCTCGGAGTCAGGTCGACCCCACGTCCGCCAACTTCCTGATCTGGCCCC
lCysLysThrArgThrValIleTyrGluIleProArgSerGlnValAspProThrSerAlaAsnPheLeuIleTrpProP
              100                                         Sal I                120
        410       420       430       440       450       460       470       480
CGTGCGTGGAGGTGAAACGCTGCACCGGCTGCTGCAACACGAGCAGTGTCAAGTGCCAGCCCTCCCGCGTCCACCACCGC
roCysValGluValLysArgCysThrGlyCysCysAsnThrSerSerValLysCysGlnProSerArgValHisHisArg
                        130                            140
        490       500       510       520       530       540       550       560
AGCGTCAAGGTGGCCAAGGTGGAATACGTCAGGAAGAAGCCAAAATTAAAAGAAGTCCAGGTGAGGTTAGAGGAGCATTT
SerValLysValAlaLysValGluTyrValArgLysLysProLysLeuLysGluValGlnValArgLeuGluGluHisLe
     150      Bal I                    160
        570       580       590       600       610    ▼ 620      170 630        640
GGAGTGCGCCTGCGCGACCACAAGCCTGAATCCGGATTATCGGGAAGAGGACACGGATGTGAGGTGAGGATGAGCCGCAG
uGluCysAlaCysAlaThrThrSerLeuAsnProAspTyrArgGluGluAspThrAspValArg
                                              PDGF A-1      190
        650      180 660       670       680       690       700       710       720
CCCTTTCCTGGGACATGGATGTACATGGCGTGTTACATTCCTGAACCTACTATGTACGGTGCTTTATTGCCAGTGTGCGG
        730       740       750       760       770       780       790       800
TCTTTGTTCTCCTCCGTGAAAAACTGTGTCCGAGAACACTCGGGAGAACAAAGAGACAGTGCACATTTGTTTAATGTGAC
        810       820       830       840       850       860       870       880
ATCAAAGCAAGTATTGTAGCACTCGGTGAAGCAGTAAGAAGCTTCCTTGTCAAAAAGAGAGAGAGAGAGAGAGAGAGAGA
                                               Hind III
        890       900       910       920       930       940       950       960
AAACAAAACCACAAATGACAAAAACAAAACGGACTCACAAAAATATCTAAACTCGATGAGATGGAGGGTCGCCCCGTGGG
        970       980       990      1000      1010      1020      1030      1040
ATGGAAGTGCAGAGGTCTCAGCAGACTGGATTTCTGTCCGGGTGGTCACAGGTGCTTTTTTGCCGAGGATGCAGAGCCTG
       1050      1060      1070      1080      1090      1100      1110      1120
CTTTGGGAACGACTCCAGAGGGGTGGTGGTGGGCTCTGCAGGGGCCCGCAGGAAGCAGGAATGTCTTGGAAACCGCCACA
                                       Pst I
       1130      1140      1150      1160      1170      1180      1190      1200
CGAACTTTAGAAACCACACCTCCTCGCGGAATTC
```

# FIG.I

FIG.4

FIG.2     FIG.3

EP 0 366 816 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 288 307 (CHIRON CORPORATION) * whole document * | 1-6 | C 12 N 15/16 C 12 P 21/02 |
| X | CHEMICAL ABSTRACTS vol. 107, no. 17, 26 October 1987; page 172, column2, Columbus, Ohio, USA; abstract no. 148388f; B. D. TONG et al.: "cDNA clones reveal differences between human glial and endothelial cell platelet-derived growth factor A-chains" & Nature, 1987, vol. 328, no. 6131, pages 619-621 | 1-6 | |
| X | CHEMICAL ABSTRACTS vol. 108, no. 17, 25 April 1988; page 184-185, Columbus, Ohio, USA, abstract no. 144478h; F. RORSMAN et al.: "Structural characterization of the human platelet-derived growth factor A-chain cDNA and gene: alternative exon usage predicts two different precursur proteins" & Mol. Cell. Biol. 1988, vol. 8, no. 2, pages 571-577 | 1-6 | |
| X | CHEMICAL ABSTRACTS vol. 109, no. 9, 29 August 1988, page 176, column 2, Columbus, Ohio, USA, abstract no. 67916r; D. T. BONTHRON et al.: "Platelet-derived growth factor A-chain: gene structure, chromosomal location, and basis for alternative mRNA splicing" & Proc. Natl. Acad. Sci U.S.A. 1988, vol. 85, no. 5, pages 1492-1496 --- -/- | 1-6 | TECHNICAL FIELDS SEARCHED (Int. Cl.5) C 12 N 15/16 C 12 P 21/02 C 12 N 15/18 |

The present search report has been drawn up for all claims

| Place of search | Date of completion of the search | Examiner |
|---|---|---|
| BERLIN | 15-06-1989 | JULIA P. |

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 259 632  (ZYMOGENETICS INC.) * abstract; page 12, lines 22-35; page 22, line 28 - page 23, line 23; page 58, lines 16-25; figure 9; claims 1,11-12 * | 1-6 | |
| A | CHEMICAL ABSTRACTS vol. 107, no. 20, 16 November 1987; page 154, column 1, Columbus, Ohio, USA, abstract no. 1 91722x; N. F. STARKSEN et al.: "Regulated expression of the platelet-derived growth factor A-chain gene in microvascular endothelial cells" & J. Biol. Chem. 1987, vol. 262, no. 30, pages 14381-14384 | 1-6 | |
| X | BIOSIS DATABASE abstract no. 32049237; T. COLLINS et al: "Cultured human vesicular endothelial cells express the a chain of platellet-derived growth factor" & J. Cell Biochem. 1986, vol. 103, no. 5, page 10A | 1-3 | TECHNICAL FIELDS SEARCHED (Int. Cl.5) |
| X | BIOSIS DATABASE abstract no. 33040050; B.D. TONG et al: "Cloning of the a-chain of PDGF and A-chain expression in human endothelial cells" & J. Cell. Biochem.suppl. 1987, vol. 0, no. 11 Part A, page 44 | 1-6 | |

The present search report has been drawn up for all claims

| Place of search | Date of completion of the search | Examiner |
|---|---|---|
| BERLIN | 15-06-1989 | JULIA P. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    .................................................................
& : member of the same patent family, corresponding
    document

EPO FORM 1503 03.82 (P0401)